Europäisches Patentamt

European Patent Office

Office européen des brevets

(19)

(11) Veröffentlichungsnummer: **0 342 434 B1**

# EUROPÄISCHE PATENTSCHRIFT

(45) Veröffentlichungstag der Patentschrift:
02.10.91 Patentblatt 91/40

(51) Int. Cl.⁵: **A61M 16/00**

(21) Anmeldenummer: 89108031.9

(22) Anmeldetag: 03.05.89

(54) Fördervorrichtung zur Versorgung eines Beatmungsgerätes mit Atemgas.

(30) Priorität: 19.05.88 DE 3817092

(43) Veröffentlichungstag der Anmeldung:
23.11.89 Patentblatt 89/47

(45) Bekanntmachung des Hinweises auf die
Patenterteilung:
02.10.91 Patentblatt 91/40

(84) Benannte Vertragsstaaten:
DE FR GB SE

(56) Entgegenhaltungen:
US-A- 4 076 021
US-A- 4 157 092
US-A- 4 243 029

(73) Patentinhaber: Drägerwerk
Aktiengesellschaft
Moislinger Allee 53-55
W-2400 Lübeck 1 (DE)

(72) Erfinder: Kiske, Siegfried, Dr. Dipl.-Ing.
Am Sonnenberg 1e
W-2401 Gross Grönau (DE)
Erfinder: Haase, Thorsten, Dipl.-Ing.
Waidmannsplatz 2
W-2410 Mölln (DE)
Erfinder: Leyer, Thomas, Dipl.-Ing.
Wendische Strasse 16
W-2400 Lübeck (DE)
Erfinder: Sauer, Wolfgang, Dipl.-Ing.
Glashüttenweg 16
W-2400 Lübeck (DE)

## Beschreibung

Die Erfindung betrifft eine Fördervorrichtung zur Versorgung eines Beatmungsgerätes mit Atemgas, mit einer auswechselbaren Kolben-Zylinder-Einheit, die einen in einem Zylinder laufenden Kolben enthält, der über eine an einem Bewegungsantrieb angreifende Kupplung mit einer Antriebseinheit zur Erzeugung von Förderhüben verbunden ist, wobei in den Zylinder innenraum Gasanschlußleitungen für das zu fördernde Atemgas münden.

Eine derartige Fördervorrichtung ist aus der US-PS 4157092 bekanntgeworden.

Diese bekannte Fördervorrichtung dient dazu, Atemgas in den Atemkreislauf eines Beatmungsgerätes zu fördern. Sie enthält eine Kolben-Zylinder-Einheit, die in das Gehäuse des Beatmungsgerätes einsetzbar, durch Schrauben verriegelbar und aus diesem auch nach Lösen der Schrauben wieder entnehmbar ist. Die Grundplatte des Kolbens trägt als Kupplungshilfe magnetisierbare Einsätze, die von Permanentmagneten berührt und festgehalten werden. Diese Magnete sind in einer Scheibe aufgenommen, welche an einem Bewegungsantrieb befestigt ist, der als mit einer Antriebseinheit verbundene Schubstange ausgebildet ist. Die Magnete nehmen den Kolben bei den Förderhüben mit und übertragen somit die Pumphübe für das Atemgas, welches über fest mit der Kolben-Zylinder-Einheit verbundene Anschlußleitungen in die Fördervorrichtung eintritt und aus ihr wieder herausgeleitet wird. Zum Auswechseln der Kolben-Zylinder-Einheit müssen zuerst die Verriegelungsschrauben gelöst werden und dann die Kolben-Zylinder-Einheit durch einfaches Herausziehen der letzteren und nach Überwinden der entgegengesetzten Magnetkraft herausgenommen.

Bei der bekannten Fördervorrichtung kann die Kolben-Zylinder-Einheit während des Betriebes der Antriebseinheit herausgenommen werden oder sich lösen, wodurch unter Umständen ungewollte Betriebsstörungen auftreten können. Beim Entnehmen erfordert das Lösen der Gasleitungen zusätzliche Handgriffe. Bei entnommener Kolben-Zylinder-Einheit ist der Kolben nicht fixiert. Beim Hantieren eintretende unkontrollierte Verschiebungen können zu Beschädigungen führen oder das spätere Ankuppeln behindern.

Der vorliegenden Erfindung liegt somit die Aufgabe zugrunde, eine Fördervorrichtung der genannten Art so zu verbessern, daß die Kolben-Zylinder-Einheit einfach zu entnehmen und unter sicherer Führung wieder einzusetzen ist, wobei eine automatische Verriegelung derart erfolgen soll, daß ein Lösen und Entnehmen der Kolben-Zylinder-Einheit aus dem Beatmungsgerät und ihre Entkupplung von der Antriebseinheit nur in einer Ruhestellung, nicht aber während des Betriebs möglich ist.

Die Lösung der Aufgabe erfolgt dadurch, daß zum Auswechseln der Kolben-Zylinder-Einheit der Bewegungsantrieb einen Bereitschaftshub ausführt, in dessen Endstellung sowohl die Kupplung als auch eine Verriegelung der Kolben-Zylinder-Einheit gegenüber dem diese aufnehmenden Gehäuse betätigbar sind.

Eine Entnahme der Kolben-Zylinder-Einheit kann nunmehr erst dann erfolgen, wenn die Fördervorrichtung absichtlich in eine Bereitschaftsstellung umgeschaltet wird, in welcher die Hubbewegung des Bewegungsantriebes in eine Verriegelungsstellung gebracht wird, in welcher die Lage des Kolbens in bezug auf den Zylinder festgelegt ist. Ein Herausnehmen und ein anschließendes Wiedereinsetzen der Kolben-Zylinder-Einheit ermöglicht deshalb ein sicheres Entkuppeln und Ankuppeln des Bewegungsantriebs mit der Antriebseinheit, so daß eine Justierung der Position des Bewegungsantriebes vor dem Wiedereinsetzen nicht erforderlich ist. Durch die Erzeugung eines Bereitschaftshubs und sein Verharren in dessen Endstellung bleibt die Position der Antriebseinheit und der zugehörigen Kupplung so lange fest, bis die Antriebseinheit nach dem Einsetzen der Kolben-Zylinder-Einheit zur Durchführung von Förderhüben erneut eingeschaltet wird.

Die Kontrolle der Hubbewegungen wird zweckmäßigerweise durch einen Wegaufnehmer registriert und an die Steuereinheit weitergegeben, so daß bei eingeschalteter Antriebseinheit die Grenzen der Förderhübe durch den Wegaufnehmer festgelegt sind. Beim Ausschalten der Antriebseinheit wird dann die Kontrolle über den Wegaufnehmer aufgehoben und der Bewegungsantrieb über einen größeren Bereitschaftshub in seine Bereitschaftsstellung gebracht.

Ein besonders sicheres und widerstandsarmes Gleiten des Kolbens in dem Zylinder wird durch eine Doppelrollmembran ermöglicht.

Zweckmäßigerweise wird bei Betätigung der Verriegelung zur Entkupplung des Bewegungsantriebes der Kolben in seiner Position arretiert und der Bewegungsantrieb stillgesetzt. Beschädigungen an beispielsweise Dichtoberflächen durch einen sonst frei beweglichen Kolben sind ausgeschlossen. Damit ist sichergestellt, daß die einmal beim Herausnehmen der Kolben-Zylinder-Einheit eingenommene Position des Kolbens relativ zu dem Zylinder auch während einer nachfolgenden Wartung, Reinigung oder Desinfektion oder sonstigen Handhabungen fixiert bleibt und er nach Durchführung der erwähnten Arbeiten wiederum in dieselbe Stellung einkuppelbar ist. Außerdem wird beim Auswechseln von verschiedenen Kolben-Zylinder-Einheiten in das Gehäuse der Fördervorrichtung sichergestellt, daß für alle Einheiten dieselbe Position für ein schnelles und sicheres Einsetzen gültig ist.

Gleichzeitig mit Betätigung der Verriegelung sind zweckmäßigerweise die Mündungen der Gasanschlußleitungen über Dichtelemente an ihre Gasversorgungsleitungen anschließbar. Somit ist die Fördervorrichtung unmittelbar nach dem Einsetzen der Kolben-Zylinder-Einheit zur Förderung des Atemgases betriebsfertig.

Zur formschlüssigen Ankupplung von Bewegungsantrieb an die Antriebseinheit ist ein Kupplungsstück mit einer Kolbenstange als Bewegungsantrieb verbunden, deren knopfartiges Ende mittels mindestens zweier unter Federzugkraft stehenden Kippklauen am Kupplungsstück hinterfaßbar sind, welche von dem Knopfende mittels Druckstiften abhebbar sind, die dazu durch die Drehbewegung einer am Kupplungsstück befestigten Kurvenscheibe, welche an ihren Angriffsflächen zu den Stiften mit einer schiefen Ebene versehen ist, der Zugkraft entgegenwirkend bewegbar sind. Das formschlüssige Hintergreifen der Kippklauen sichert eine während des Förderhubes starre Verbindung zwischen Kolbenstange und Antriebseinheit, ist jedoch in der Lage, bei ruckartigen Antriebsbewegungen die Kolbenstange so weit freizugeben, daß die Ruckbewegungen geglättet und nicht über das Atemgas auf den Patienten übertragen werden ; gleichwohl bleibt die Federkraft, mit denen die Kippklauen das Knopfende hintergreifen, ausreichend, um den nachfolgenden gleichförmigen Hubbewegungen zu folgen. Ist der Bereitschaftshub erreicht, geben die Kippklauen das Knopfende frei, sobald durch die Drehbewegung der Kurvenscheibe die Druckstifte die Kipphebel von dem Knopfende abdrücken.

Zweckmäßigerweise ist an der Kolben-Zylinder-Einheit eine über einen Schwenkgriff betätigbare Schrägscheibe angebracht, die einen Schaltstift besitzt, der beim Einsetzen der Kolben-Zylinder-Einheit in das Gehäuse der Fördervorrichtung in eine Antriebsnut der Kurvenscheibe eingreift. Beim Schwenken des Griffes werden die Verbindung von Kupplungsstück und Kolbenstange sowie die Verbindung der pneumatischen Schnittstellen zwischen den Gasverbindungsanschlüssen und ihren Gasversorgungsleitungen hergestellt. Das Schwenken des Griffes dreht die Kurvenscheibe, dabei wird die schiefe Ebene gegen die Angriffsflächen der Druckstifte geführt, wodurch die Kippklauen von dem Knopfende abgehoben werden (Entriegelung). Beim Zurückschwenken des Griffes wird die schiefe Ebene von den Angriffsflächen der Druckstifte entfernt, so daß die Kippklauen wiederum unter Federzugkraft gebracht werden und hinter das Knopfende der Kolbenstange greifen. (Verriegelung).

Zweckmäßigerweise trägt der Schwenkgriff an seiner Schwenkachse eine mit einer Rastausnehmung versehenen Schrägscheibe. In die Rastausnehmung greift eine Raste ein, welche in der Endstellung des Bereitschaftshubes aus der Rastausnehmung entfernbar ist. Während der Förderhübe greift die Raste in die Rastausnehmung ein und verhindert ein Betätigen des Schwenkgriffes und somit ein ungewolltes Entkuppeln der Kolben-Zylinder-Einheit von der Antriebseinheit während des Betriebs. Erst in der Endstellung des Bereitschaftshubes ist es möglich, die Raste aus der Rastausnehmung zu entfernen und die Kolben-Zylinder-Einheit zu entkuppeln.

Es ist günstig, die Raste als eine federelastische Zunge auszubilden, welche in die Rastausnehmung eingreift, und die Kolbenstange mit einer Verdickung zu versehen, durch die bei Durchführen des Bereitschaftshubes die Zunge aus der Rastausnehmung herausgedrückt wird. Somit ist eine automatische Freigabe des Schwenkgriffes zur Entkupplung der Kolben-Zylinder-Einheit von der Antriebseinheit gegeben, sobald der Bereitschaftshub beendet ist.

Um bei herausgenommener Kolben-Zylinder-Einheit eine relative Bewegung des Kolbens zu dem Zylinder zu vermeiden und ein nachträgliches Einsetzen und Ankuppeln zu vereinfachen, ist eine Hubsperre vorgesehen. Dazu ist die Kolbenstange mit einer Sperrausnehmung versehen, in welche in der Endstellung des Bereitschaftshubes bei herausgenommener Kolben-Zylinder-Einheit ein Sperrschieber eingreift. Der Sperrschieber ist zur Sperrstellung federbelastet und bei eingesetzter Kolben-Zylinder-Einheit mit einem Auslösestift in Eingriff. Der Auslösestift entriegelt die Hubsperre, so daß dann zum Betrieb die Arbeitshübe durchführbar sind.

Ein günstiger Anbringungsort für den Schwenkgriff besteht darin, ihn stirnseitig zum Zylinder anzusetzen und an seiner radial zum Zylinder versetzten Schwenkachse mit einer Schrägscheibe zu versehen. Bei Betätigung des Schwenkgriffes zur Ankupplung des Bewegungsantriebes an die Antriebseinheit wird durch die Taumelbewegung der Schrägscheibe die Kolben-Zylinder-Einheit um eine Stütze gekippt, und die am äußeren Umfang des Zylinders in der Kippebene liegenden Mündungen der Gasanschlußleitungen werden auf die zugehörigen Gasversorgungleitungen gedrückt. Die Kippbewegung wird dadurch ermöglicht, daß die Schrägscheibe bei Betätigung des Schwenkgriffes gegen ein Druckstück gepreßt wird, wodurch die Kolben-Zylinder-Einheit eine Kippbewegung um die Stütze als Kippunkt ausführt und die in der Kippebene liegenden Anschlußöffnungen gegen eine Lippendichtung preßt. Damit ist eine leckfreie Abdichtung der Anschlußleitungen bei verriegelter Kolben-Zylinder-Einheit möglich.

Ein Wegaufnehmer nimmt die Endposition des Förderhubes auf und schaltet durch eine Steuereinheit den Ablauf der Förderhübe. Erst durch beabsichtigtes Übergehen der Kontrolle durch den Wegaufnehmer, z.B. durch eine Sonderschaltung an der Steuereinheit, kann ein Bereitschaftshub ausgeführt werden.

Ein Ausführungsbeispiel der Erfindung wird anhand der schematischen Zeichnung dargestellt und im folgenden näher erläutert.

Es zeigen :

EP 0 342 434 B1

Figur 1    eine Seitenansicht im Teilschnitt durch die Kolben-Zylinder-Einheit in Förderhubstellung ;
Figur 2    eine Draufsicht auf das Kupplungsstück mit Antriebseinheit für die Kolbenstange ;
Figur 3    die Ansicht auf die Schrägscheibe mit der Rastausnehmung, der Raste und der Verriegelung.

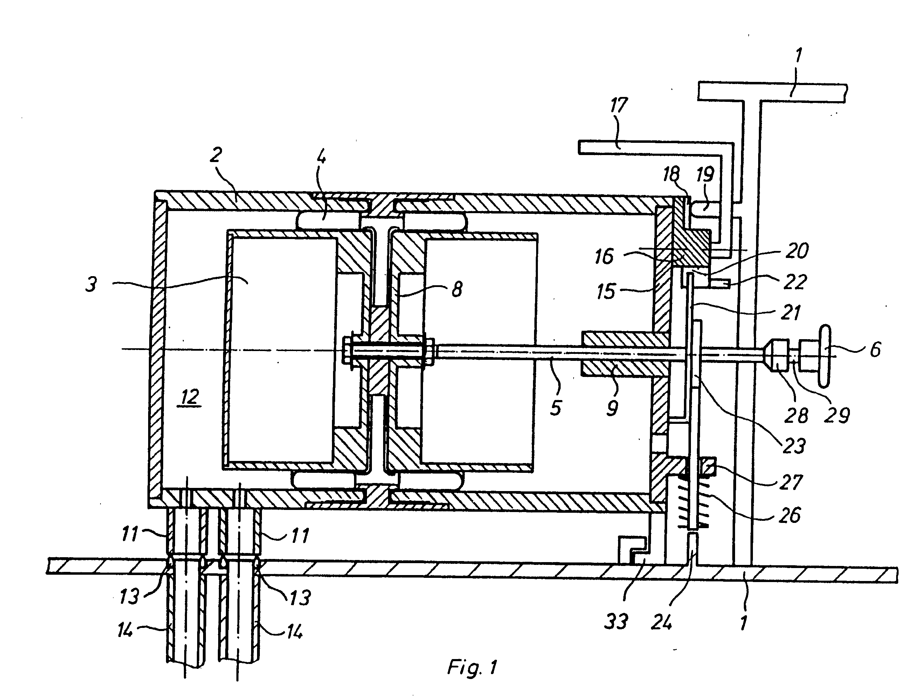

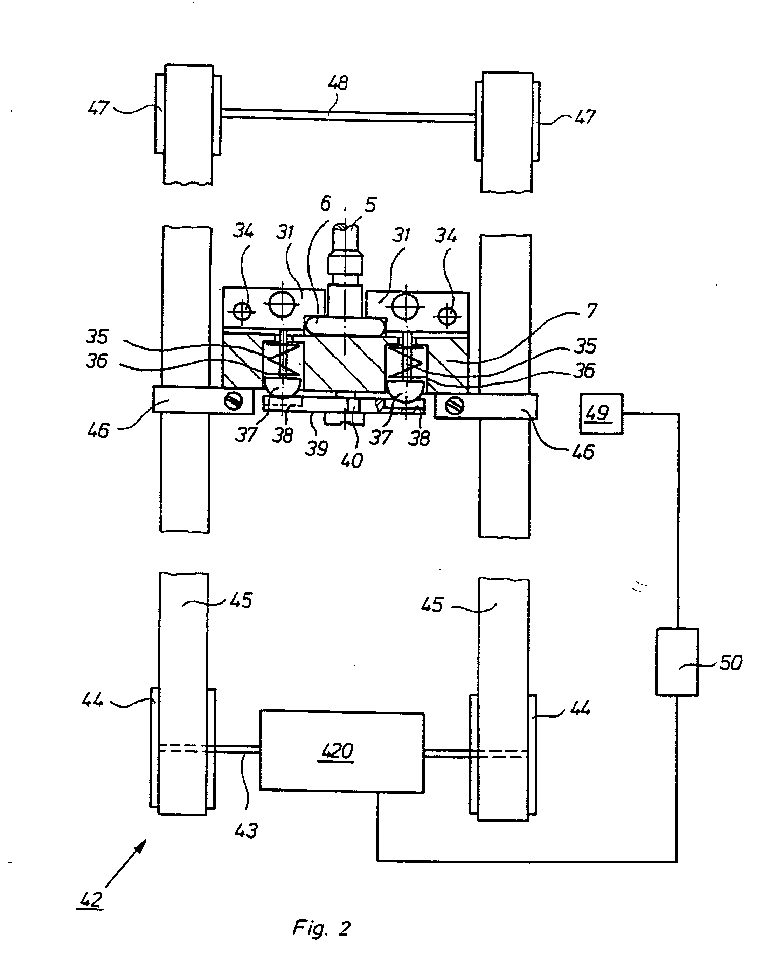

In Figur 1 ist die Kolben-Zylinder-Einheit im Schnitt dargestellt, wie sie auf dem Gehäuse (1) eines Beatmunsgerätes eingesetzt ist. Das Gehäuse (1) ist nicht vollständig dargestellt, sondern beinhaltet lediglich diejenigen Elemente, welche zur Erklärung des Beispieles erforderlich sind. Die Hauptbestandteile der Kolben-Zylinder-Einheit sind der Zylinder (2), in welchem ein Kolben (3) über eine Doppelrollmembran (4) aufgenommen ist. Die Hubbewegungen für den Kolben (3) werden durch einen als Kolbenstange (5) ausgebildeten Bewegungsantrieb von einer in Figur 2 dargestellten Antriebseinheit (42) erzeugt. Die Antriebseinheit (42) greift an einem Knopfende (6) der Kolbenstange (5) mittels eines Kupplungsstückes (7) an, welches näher in Figur 2 dargestellt ist. Die Kolbenstange (5) ist an einem Zwischenboden (8) des Kolbens befestigt und in einer Buchse (9) verschiebbar gelagert. Der Kolben (3) schließt im Innenraum des Zylinders (2) eine mit Gasanschlußleitungen (11) versehene Arbeitskammer (12) ein. Die Gasanschlußleitungen (11) sind über eine Lippendichtung (13) mit den dazugehörigen Gasversorgungsleitungen (14) verbunden. Die die Buchse (9) tragende Abschlußplatte (15) des Zylinders (2) besitzt eine Schrägscheibe (16), an welche ein Schwenkgriff (17) angesetzt ist. Die Schrägscheibe (16) weist einerseits eine Schrägfläche (18) auf, die gegenüber einem am Gehäuse (1) befestigten Druckstück (19) anliegt und zeigt andererseits eine Rastausnehmung (20), in welche eine Raste (21) eindringt. Außerdem ragt parallel zur Drehachse der Schrägscheibe (16) ein Schaltstift (22) heraus. Weiterhin ist stirnseitig zur Abschlußplatte (15) ein Sperrschieber (23) angeordnet, welcher die Kolbenstange (5) bei der dargestellten Förderhubstellung der Kolben-Zylinder-Einheit (2,3) berührungslos umfaßt. Ein Auslösestift (24) im Gehäuse (1) hält bei eingesetztem Zylinder (2) den Sperrschieber (23) in dieser Lage. Eine Sperrfeder (26) stützt sich gegen einen Halter (27) ab, durch den der Sperrschieber (23) hindurchgeführt ist. Die Raste (21) umschließt ebenfalls die Kolbenstange (5), ohne daß die Hubbewegung von der Raste (21) behindert wird. In unmittelbarer Nähe zum Knopfende (6) befindet sich auf der Kolbenstange (5) eine Verdickung (28) sowie eine Sperrausnehmung (29).

Während eines Förderhubes gleitet, angetrieben von der Antriebseinheit (42) über das Kupplungsstück (7), die Kolbenstange (5) in der Buchse (9) sowie durch die Raste (21) und den Sperrschieber (23) hin und her. Die Hubbewegungen des Kolbens (3) werden durch das Abwälzen der den Umfang des Kolbens (3) umschließenden Doppelrollmembran (4) auf der Innenfläche des Zylinders (2) ermöglicht. Der Schwenkgriff (17) ist durch die in der Rastausnehmung (20) eingerastete Raste (21) in seiner Schwenkbewegung blockiert. Dadurch ist ein Entnehmen der Kolben-Zylinder-Einheit (2,3) vom Gehäuse (1) nicht möglich, denn das auf der Schrägfläche (18) der Schrägscheibe (16) ruhende Druckstück (19) gibt keine Bewegungsfreiheit, um die in der Hakenstütze (33) verankerte Kolben-Zylinder-Einheit zu entfernen. Zugleich bewirkt der auf die Schrägfläche (18) der Schrägscheibe (16) ausgeübte Druck ein Kippmoment um die Hakenstütze (33), so daß die Gasanschlußleitungen (11) fest auf den Lippendichtungen (13) abgestützt sind.

In Figur 2 ist das Kupplungsstück (7) dargestellt, welches mit seinen Kippklauen (31) das Knopfende (6) der Kolbenstange (5) umgreift. Die Kippklauen (31) sind in Gelenken (34) aufgenommen und stehen jeweils mit einem Druckstift (35) in Verbindung. Die Druckstifte (35) sind in der dargestellten Position der Druckkraft einer Druckfeder (36) ausgesetzt und ergeben somit eine Klemmwirkung der Kippklauen (31) auf das Knopfende (6). Jeder Stiftkopf (37) liegt gegenüber einer schiefen Ebene (38), welche in eine drehbare Kurvenscheibe (39) eingelassen ist. Die Kurvenscheibe (39) ist mit einer Antriebsnut (40) versehen, in die der Schaltstift (22) der Schrägscheibe (16) eingreifen kann. Die Hubbewegungen werden innerhalb einer Antriebseinheit (42) von einem Antriebsmotor (420) über seine Antriebsachse (43) an die Antriebsräder (44) und von dort mittels Zahnriemen (45) auf das Kupplungsstück (7) übertragen. Die Befestigung des Kupplungsstückes (7) an die Zahnriemen (45) erfolgt mit Schraubklemmen (46). Die Zahnriemen (45) laufen über zwei Laufräder (47) an ihrer Laufachse (48) zu den Antriebsrädern (44) zurück. Die Position eines Förderhubes wird über eine Lichtschranke (49) als Wegaufnehmer überwacht, welche ein Positionssignal an eine Steuereinheit (50) weitergibt, durch welche die Antriebseinheit in Geschwindigkeit und Drehsinn angesteuert wird.

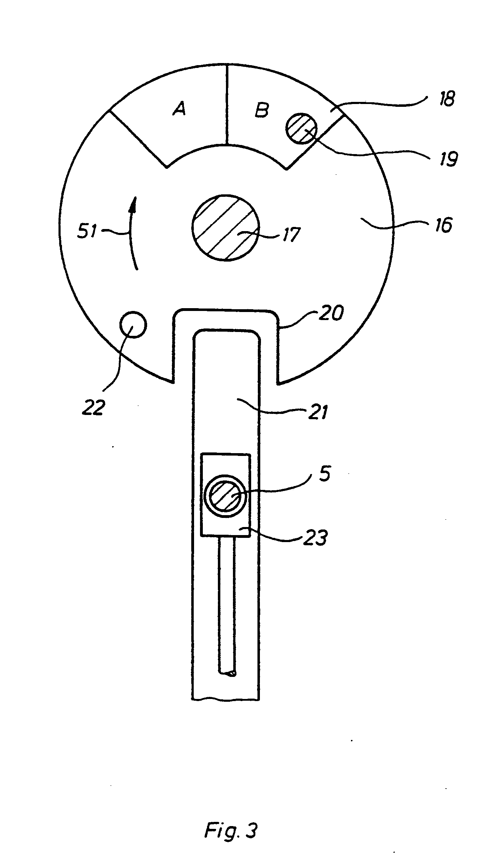

In Figur 3 ist die Ansicht der Schrägscheibe (16), gesehen in Richtung der Abschlußplatte (15), dargestellt. Sie besitzt an ihrem oberen Umfang die Schrägfläche (18), die sich in eine vertiefte Teilfläche A und eine angeschrägte, erhöhte Teilfläche B unterteilt. Am unteren Umfang der Schrägscheibe (16) befindet sich die Rastausnehmung (20), in welche die Raste (21) eingreift. Aus der Schrägscheibe (16) ragt der Schaltstift (22) heraus. Die Kolbenstange (5) ist von dem Sperrschieber (23) umgeben, in dem eine Bohrung von solchem Durchmesser vorgesehen ist, daß sowohl die Kolbenstange (3) als auch die Verdickung (28) hindurchpaßt. Die Raste (21) besitzt ebenfalls eine Bohrung, durch die zwar die Kolbenstange (5), nicht jedoch die Verdickung (28) hin-durchpaßt.

4

Ein Entkuppeln der Kolben-Zylinder-Einheit von der Antriebseinheit (42) wird wie folgt ausgeführt: Zunächst führt die Kolbenstange (5) einen Bereitschaftshub aus, welcher beispielsweise durch einen entsprechenden Wahlschalter an der Steuereinheit (50) ausgelöst werden kann. Dabei wird das Knopfende (6) von dem Kupplungsstück (7) so weit in Richtung der Arbeitskammer (12) vorgeschoben, daß die Verdickung (28) durch den Sperrschieber (23) hindurch auf die Raste (21) trifft und diese aus der Rastausnehmung (20) aushebt. Gleichzeitig faßt der Schaltstift (22) in die Antriebsnut (40). Dabei bleibt der Sperrschieber (23) außerhalb der Sperrausnehmung (29). Durch Schwenken des Schwenkgriffes (17) wird nun einerseits die an dem Druckstück (19) aufliegende Teilfläche B der Schrägfläche (18) verschoben, so daß nunmehr die von dem Druckstück (19) freistehende Teilfläche A dem Druckstück (19) gegenübersteht, andererseits wird die Kurvenscheibe (39) an ihrer Antriebsnut (40) durch den Schaltstift (22) aus ihrer in Figur 2 dargestellten Endlage in ihre andere Endlage geschwenkt, so daß ihre schiefen Ebenen (38) auf die Stiftköpfe (37) auflaufen. Die Druckstifte (35) werden entgegen der Federkraft der Druckfedern (36) bewegt, so daß die Kippklauen (31) das Knopfende (6) freigeben. Die Kolben-Zylinder-Einheit (2,3) kann jetzt mittels des Schwenkgriffes (17) aus dem Gehäuse (1) entnommen werden. Dabei wird der Sperrschieber (23) von dem Auslösestift (24) entfernt, so daß die Sperrfeder (26) den Sperrschieber (23) in die Sperrausnehmung (29) zieht. Damit ist die Position der Kolbenstange (5) festgehalten.

Beim Wiedereinsetzen und Verriegeln sowie Ankuppeln der Zylinder-Kolben-Einheit (2,3) an die Antriebseinheit (42) wird das Knopfende (6) zwischen die offenstehenden Kippklauen (31) gesetzt. Gleichzeitig faßt der Schaltstift (22) in die Antriebsnut (40), der Sperrschieber (23) wird durch den Auslösestift (24) angehoben und aus dem Eingriff mit der Sperrausnehmung (29) gebracht, so daß die Kolbenstange (5) freigegeben wird. Gleichzeitig wird die Schrägscheibe (16) in Sperrstellung gebracht, in der die Teilfläche B der Schrägfläche (18) gegen das Druckstück (19) gedreht wird und somit die gesamte Kolben-Zylinder-Einheit um die Hakenstütze (33) gekippt und die Gasanschlußleitungen (11) gegen ihre Lippendichtung (13) angepreßt werden. Gleichzeitig mit der Drehbewegung der Schrägscheibe legt der Schaltstift (22) die Kurvenscheibe (39) in ihre Verriegelungsstellung um, wodurch ihre schiefen Ebenen (38) die Stiftköpfe (37) der Druckstifte (35) freigeben, so daß diese unter der Druckwirkung ihrer Druckfedern (36) die Kippklauen (31) gegen das Knopfende (6) drücken, welches somit auf der Auflagefläche des Kupplungsstückes (7) anliegt. Bei dem gesamten bis jetzt genannten Vorgang der Verriegelung greift die Raste (21) noch nicht in die Rastausnehmung (20), sondern dies wird erst durch den ersten Förderhub in Richtung Abschlußplatte (15) bewirkt, weil erst dann die Verdickung (28) an der Kolbenstange (5) von der Raste (21) entfernt wird und diese in die Rastausnehmung (20) zurückspringt. Jetzt ist die Schwenkscheibe blockiert und ein Lösen und Herausnehmen der Kolben-Zylinder-Einheit (2,3) während der Förderhübe nicht mehr möglich.

Alle Handgriffe zur Verriegelung, Kupplung und Entnahme der Kolben-Zylinder-Einheit (2,3) sind alleine durch Betätigung des Schwenkgriffes (17) durchführbar. Die übrigen Funktionen erfolgen zwangsläufig.

## Patentansprüche

1. Fördervorrichtung zur Versorgung eines mit einer auswechselbaren Kolben-Zylinder-Einheit (2, 3), die Beatmungsgerätes mit Atemgas, einen in einem Zylinder (2) laufenden Kolben (3) enthält, der über eine an einem Bewegungsantrieb (5) angreifende Kupplung (7) mit einer Antriebseinheit zur Erzeugung von Förderhüben verbunden ist, wobei in den Zylinderinnenraum Gasanschlußleitungen (14) für das zu fördernde Atemgas münden, dadurch gekennzeichnet, daß zum Auswechseln der Kolben-Zylinder-Einheit (2, 3) der Bewegungsantrieb (5) einen Bereitschaftshub ausführt, in dessen Endstellung sowohl die Kupplung (7) als auch eine Verriegelung (20, 21) der Kolben-Zylinder-Einheit (2, 3) gegenüber dem diese aufnehmenden Gehäuse (1) betätigbar sind.

2. Fördervorrichtung nach Anspruch 1, dadurch gekennzeichnet, daß der Kolben (3) gegenüber dem Zylinder (2) mit einer Doppelrollmembran (4) abgedichtet ist.

3. Fördervorrichtung nach Anspruch 1 oder 2, dadurch gekennzeichnet, daß der Kolben (3) bei Betätigung der Verriegelung (20, 21) und der Kupplung (7) durch eine Hubsperre (23, 29) arretierbar ist.

4. Fördervorrichtung nach einem der Ansprüche 1 bis 3, dadurch gekennzeichnet, daß die Mündungen der Gasanschlußleitungen (11) durch Betätigung der Verriegelung (20, 21) über Dichtelemente (13) an ihre Gasversorgungsleitungen (14) anschließbar sind.

5. Fördervorrichtung nach einem der Ansprüche 1 bis 4, dadurch gekennzeichnet, daß die Antriebseinheit (42) über ein Kupplungsstück (7) mit einer Kolbenstange (5) als Bewegungsantrieb verbunden ist, deren knopfartiges Ende (6) mittels mindestens zweier am Kupplungsstück (7) angeordneten, unter Federzugkraft stehenden Kippklauen (31) hinterfaßbar ist, welche von dem Knopfende (6) mittels Druckstiften (35) abhebbar sind, die dazu durch die Drehbewegung einer am Kupplungsstück (7) befestigten Kurvenscheibe (39), welche an ihren Angriffsflächen zu den Stiften (35) mit einer schiefen Ebene (38) versehen ist, der Zugkraft entgegenwirkend bewegbar sind.

6. Fördervorrichtung nach Anspruch 5, dadurch gekennzeichnet, daß die Kurvenscheibe (39) mit einer Antriebsnut (40) versehen ist, in welche ein Schaltstift (22) einer an der Kolben-Zylinder-Einheit (2, 3) befestigten Schrägscheibe (16) eingreift, die mit einem Schwenkgriff (17) betätigbar ist.

7. Fördervorrichtung nach Anspruch 6, dadurch gekennzeichnet, daß die Schrägscheibe (16) eine Verriegelung in Gestalt einer Rastausnehmung (20) besitzt, in welche eine Raste (21) eingreift, die in der Endstellung des Bereitschaftshubes aus der Rastausnehmung (20) entfernbar ist.

8. Fördervorrichtung nach Anspruch 7, dadurch gekennzeichnet, daß an der Kolbenstange (5) eine Verdickung (28) vorgesehen ist, durch die bei Durchführung des Bereitschaftshubes die die Kolbenstange (5) umgreifende, als federelastische Zunge ausgebildete Raste (21) aus der Rastausnehmung (20) ausrastbar ist.

9. Fördervorrichtung nach einem der Ansprüche 1 bis 8, dadurch gekennzeichnet, daß in der Endstellung des Bereitschaftshubes bei herausgenommener Kolben-Zylinder-Einheit (2, 3) als Hubsperre (23, 29) ein zur Sperrstellung federbelasteter Sperrschieber (23) in eine Sperrausnehmung (29) des Bewegungsantriebs (5) eingreift, wobei der Sperrschieber (23) bei eingesetzter Kolben-Zylinder-Einheit (2, 3) mit einem Auslösestift (24) in Eingriff ist, durch den der Sperrschieber (23) durch eine entgegen der Federkraft gerichtete Bewegung aus der Sperrausnehmung (29) entfernbar ist.

10. Fördervorrichtung nach einem der Ansprüche 6 bis 9, dadurch gekennzeichnet, daß die Schrägscheibe (16) mit einer Schrägfläche (18) versehen ist, durch die bei Betätigung des Schwenkgriffes (17) zur Ankupplung des Bewegungsantriebes (5) an das Kupplungsstück (7) die Kolben-Zylinder-Einheit (2, 3) um eine Stütze (33) kippbar ist, wobei die Mündungen der Gasanschlußleitungen (11) am äußeren Umfang des Zylinders (2) einerseits und andererseits sowohl in der Kippebene liegend als auch den Gasversorgungsleitungen (14) gegenüberstehend angebracht sind.

11. Fördervorrichtung nach einem der Ansprüche 1 bis 10, dadurch gekennzeichnet, daß zur Kontrolle der Förderhübe ein Wegaufnehmer (49) vorgesehen ist.

## Claims

1. Conveying arrangement for the supply of a respiratory apparatus with respiratory gas, having a replaceable piston-cylinder unit (2, 3) which contains a piston (3) running in a cylinder (2) and connected, by way of a coupling (7) acting on a motion drive (5), with a drive unit for the purpose of generating conveying strokes, with gas connection lines (14) for the respiratory gas to be conveyed running into the interior space of the cylinder, characterised in that for the purpose of replacing the piston-cylinder unit (2, 3) the motion drive (5) executes a stand-by stroke in the end position of which both the coupling (7) and a locking device (20, 21) of the piston-cylinder unit (2, 3) can be actuated in relation to the housing (1) accommodating the latter.

2. Conveying arrangement according to claim 1, characterised in that the piston (3) is sealed in relation to the cylinder (2) with a double rolling membrane (4).

3. Conveying arrangement according to claim 1 or 2, characterised in that the piston (3) can be arrested upon actuation of the locking device (20, 21) and the coupling (7) by means of a stroke block (23, 29).

4. Conveying arrangement according to one of the claims 1 to 3, characterised in that the mouths of the gas connection lines (11) can be connected to their gas supply lines (14) by way of sealing elements (13) by actuation of the locking device (20, 21).

5. Conveying arrangement according to one of the claims 1 to 4, characterised in that the drive unit (42) is connected via a coupling piece (7) with a piston rod (5) as a motion drive, behind the button-like end (6) of which there can grip at least two tilting claws (31) which are arranged on the coupling piece (7), are under spring tensile force and can be lifted off from the button end (6) by means of pressure pins (35) which can be moved in opposition to the tensile force for this purpose as a result of the rotational movement of a cam disc (39) which is secured to the coupling piece (7) and is provided with an inclined plane (38) on its surfaces of action in respect of the pins (35).

6. Conveying arrangement according to claim 5, characterised in that the cam disc (39) is provided with a drive groove (40) into which there engages an operating pin (22) of a chamfered disc (16) which is secured to the piston-cylinder unit (2, 3) and can be actuated with a swing handle (17).

7. Conveying arrangement according to claim 6, characterised in that the chamfered disc (16) has a locking device in the form of a stop recess (20) into which there engages a stop (21) which can be removed from the stop recess (20) in the end position of the stand-by stroke.

8. Conveying arrangement according to claim 7, characterised in that there is provided on the piston rod (5) a thickened portion (28) by means of which, when the stand-by stroke is carried out, the stop (21), which encompasses the piston rod (5) and is formed as a spring-elastic tongue, can be released from the stop recess (20).

9. Conveying arrangement according to one of the claims 1 to 8, characterised in that in the end position of the stand-by stroke, with piston-cylinder unit (2, 3) removed, as the stroke block (23, 29) a blocking slide (23), which is spring-loaded in respect of the blocking position, engages into a blocking recess (29) of the motion drive (5), with the blocking slide (23) being engaged with a release pin (24) when the piston-cylinder unit (2, 3) is inserted, by means of which pin the blocking slide (23) can be removed from the blocking recess (29) by means of a movement directed in opposition to the spring force.

10. Conveying arrangement according to one of the claims 6 to 9, characterised in that the chamfered disc (16) is provided with a chamfered surface (18) by means of which upon actuation of the swing handle (17) for the purpose of coupling the motion drive (5) to the coupling piece (7) the piston-cylinder unit (2, 3) can be tilted about a support (33), the mouths of the gas connection lines (11) being provided on the outer periphery of the cylinder (2), on the one hand, and, on the other hand, not only lying in the plane of tilt, but also standing opposite the gas supply lines (14).

11. Conveying arrangement according to one of the claims 1 to 10, characterised in that a position sensor (49) is provided for the purpose of controlling the conveying strokes.


**Revendications**

1. Dispositif de transport pour l'alimentation d'un respirateur, en gaz de respiration, avec une unité à cylindre et piston (2, 3) interchangeable qui contient un piston (3) se déplaçant dans un cylindre (2), lequel est relié, par l'intermédiaire d'un accouplement (7) agissant sur un mécanisme d'entraînement en déplacement (5), à une unité d'entraînement, en vue d'engendrer des courses de transport, des conduits de raccord de gaz (14) pour le gaz de respiration à transporter, débouchant à l'intérieur du cylindre, caractérisé en ce que pour changer l'unité à cylindre et piston (2, 3), le mécanisme d'entraînement en déplacement (5) exécute une course de préparation à la fin de laquelle l'accouplement (7) ainsi qu'un verrouillage (20, 21) de l'unité à cylindre et piston (2, 3) peuvent être actionnés par rapport au boîtier (1) logeant celle-ci.

2. Dispositif de transport selon la revendication 1, caractérisé en ce que le piston (3) est rendu étanche par rapport au cylindre (2), avec une membrane à double rouleau (4).

3. Dispositif de transport selon la revendication 1 ou 2, caractérisé en ce que le piston (3) peut être bloqué par un dispositif de blocage de course (23, 29), lors de l'actionnement du verrouillage (20, 21) et de l'accouplement (7).

4. Dispositif de transport selon l'une des revendications 1 à 3, caractérisé en ce que les sorties des conduits de raccord de gaz (11) peuvent être raccordées à leurs conduits d'alimentation en gaz (14), par l'intermédiaire d'éléments d'étanchéité (13), par actionnement du verrouillage (20, 21).

5. Dispositif de transport selon l'une des revendications 1 à 4, caractérisé en ce que l'unité d'entraînement (42) est reliée par une pièce d'accouplemnt (7) à une tige de piston (5), servant de mécanisme d'entraînemnt en déplacement dont l'extrémité (6) en forme de bouton est saisie, de l'arrière, par au moins deux griffes de basculement (31), soumises à la force de traction d'un ressort, placées sur la pièce d'accouplement 7, lesquelles griffes peuvent être relevées de l'extrémité en bouton (6) au moyen de doigts de pression (35) qui à cet effet sont déplaçables à l'encontre de la force de traction, par la rotation d'un disque à cames (39), fixé sur la pièce d'accouplement (7), lequel disque à cames est pourvu d'un plan incliné (38), sur ses surfaces d'attaque des doigts (35).

6. Dispositif de transport selon la revendication 5, caractérisé en ce que le disque à cames (39) est pourvu d'une rainure d'entraînement (40) dans laquelle s'engage un doigt de commutation (22) d'un plateau oscillant (16) fixé sur l'unité à cylindre et piston (2, 3), lequel plateau peut être actionné par une poignée pivotante (17).

7. Dispositif de transport selon la revendication 6, caractérisé en ce que le plateau oscillant (16) possède un verrouillage sous la forme d'un évidement d'arrêt (20) dans lequel s'engage un cliquet (21) qui, dans la position de fin de course de préparation, peut être éloigné de l'évidement d'arrêt (20).

8. Dispositif de transport selon la revendication 7, caractérisé en ce qu'il est prévu sur la tige de piston (5) une surépaisseur (28) par laquelle le cliquet (21), en forme de languette élastique, entourant la tige de piston (5), peut être dégagé de l'évidement d'arrêt (20), pendant l'exécution de la course de préparation.

9. Dispositif de transport selon l'une des revendications 1 à 8, caractérisé en ce qu'en position de fin de course de préparation, alors que l'unité à cylindre et piston (2, 3) est extraite, un curseur de blocage (23), servant de dispositif de blocage de course (23, 29), soumis à l'action d'un ressort vers la position de blocage, s'engage dans un évidement de blocage (29) du dispositif d'entraînement en déplacement (5), le curseur de blocage (23) étant en prise avec un doigt de déclenchement (24) lorsque l'unité à cylindre et piston (2, 3) est en place, ce doigt de déclenchement permettant d'éloigner le curseur de blocage (23) de l'évidement de blocage (29), par un déplacement dirigé à l'encontre de la force du ressort.

10. Dispositif de transport selon l'une des revendications 6 à 9, caractérisé en ce que le plateau oscillant (16) est pourvu d'une surface oblique (18) par laquelle l'unité à cylindre et piston (2, 3) peut basculer autour d'un appui (33), lors de l'actionnement de la poignée de pivotement (17) en vue d'accoupler le mécanisme d'entraînement en déplacement (5) à la pièce d'accouplement (7), les sorties des conduits de raccord de gaz (11) étant appliquées sur le pourtour extérieur du cylindre (2) d'une part et d'autre part dans le plan de basculement ainsi que face aux conduits d'alimentation de gaz (14).

11. Dispositif de transport selon l'une des revendications 1 à 10, caractérisé en ce qu'il est prévu un capteur de distance (49) pour contrôler la course de transport.

*Fig. 1*

EP 0 342 434 B1

Fig. 2

*Fig. 3*